# EUROPEAN PATENT APPLICATION

(11) **EP 3 533 432 A2**
(43) Date of publication of application: **04.09.2019**
(21) Application number: 19158443.2
(22) Date of filing: 21.02.2019
(51) Int. Cl.: A61H 3/00, A61H 3/04, A61H 1/02

(54) **WEARABLE ASSISTIVE DEVICE COMPRISING FOOT SUPPORT FOR FREE ANKLE MOVEMENT**

(30) Priority: 22.02.2018 KR 20180021089; 22.02.2018 KR 20180021090; 12.06.2018 KR 20180067662; 12.09.2018 US 201862730399 P; 12.09.2018 US 201862730400 P; 12.09.2018 US 201862730412 P; 12.09.2018 US 201862730420 P
(71) Applicant: LG Electronics Inc., SEOUL (KR)
(72) Inventor: LEE, Wonjun, 08592 Seoul (KR); NAM, Bohyun, 08592 Seoul (KR); PARK, Kyu Tae, 08592 Seoul (KR); SON, Jung Kyu, 08592 Seoul (KR); YU, Seonil, 08592 Seoul (KR)
(74) Representative: Vossius & Partner Patentanwälte Rechtsanwälte mbB

(57) **Abstract**

A wearable assistive device such as an exoskeleton may include a foot support capable of a free ankle movement. The foot support may include an ankle support having an accordion spring and may be connected to a rear surface of the foot support, thereby supporting an ankle so that a user can exercise a complex ankle movement.

## Description

### BACKGROUND

### 1. Field

This application relates to assistive and/or rehabilitative technology.

### 2. Background

In assistive and/or rehabilitative technology, an assistive device such as a wearable robot or a robotic exoskeleton having a multi-joint skeletal structure may play a role in assisting or rehabilitating a user's strength or power when the user wears the wearable assistive device on his or her body. The user may wear the exoskeleton on an upper body, lower body, or an entire body. For example, for a lower body exoskeleton, the exoskeleton may assist with a walking action of the user by providing an assistive force generated from a driving apparatus such as a motor, for example, to the user.

This wearable assistive device may be secured at a waist, a leg, and/or the shoes (or the foot) of the user. A detachment mechanism that secures a body of the user to the wearable assistive device is an important factor that determines the speed, ease, and/or convenience of the device by the user. Various devices that help a user to walk are shown in U.S. Patent No. 9,956,107, U.S. Patent Application No. 2015-0182366, and KR Patent No. 10-1363834.

FIG. 1 is a view illustrating a conventional foot assistive apparatus shown in US Patent No. 9,956,107. Referring to FIG. 1, the foot assistive apparatus may include a leg supporting portion or leg support 11 to fix to a calf, a foot supporting portion or foot support 14 that supports a foot and an ankle, and a connecting frame 13 to connect the leg support 11 and the foot support 14.

The foot support 14 may include a foot section 18 that supports a foot, an ankle section 17 that supports an ankle, and a wing section 19 that covers an Achilles tendon. Such a foot assistive apparatus may assist a user in walking. The connecting frame 13 of the conventional foot assistive apparatus may be made of a rigid material, and the user may not freely move his or her ankle while wearing the foot assistive apparatus.

In addition, the foot assisting apparatus may not properly absorb an impact with the floor when the user walks while wearing the foot assistive apparatus. When the impact of the foot assistive apparatus and the floor is delivered to the user substantially unchanged, it may be difficult to provide effective rehabilitation treatment due to a continuous impact.

FIGs. 2A and 2B are views illustrating a conventional walking assistive apparatus shown in U.S. Patent application No. 2015-0182366. Referring to FIG. 2, the walking assistive apparatus discloses a joint structure that assists a free bending of a knee joint. The walking assistive apparatus may include an upper frame 56 and a lower frame 57 that are arranged at a side of a thigh and a calf, respectively, and a knee joint structure 65 connecting the upper frame 56 and the lower frame 57.

The knee joint structure 65 may include a zigzag structure 68 arranged between a first member 66 and a second member 67, and an elastic member 70 arranged between the zigzag structure 68. The zigzag structure 68 may be formed of a plurality of fragment members 68a, 68b, and 68c and a supporting shaft 69 connecting the members 68a, 68b and 68c. The knee joint structure 65 can allow a knee joint of the user to freely move while wearing a walking assistive apparatus.

Since the zigzag structure 68 may only rotate in one direction, it is difficult to support an ankle moving in forward, rearward, leftward, rightward, upward, and downward directions (i.e., a three-dimensional direction). Further, since the knee joint structure 65 is made of a highly rigid metal or resin, it fails to properly absorb an impact between the shoes and the floor. Since the lower frame 57 is provided on a side of a leg of the user, there may be interference with an ankle bone or malleolus of the user. In this case, the user may feel uncomfortable due to a collision between the ankle bone and the lower frame 57 while walking.

FIG. 3 is a view illustrating a conventional ankle joint assistive apparatus of Korean Patent No. 10-1363834. The ankle joint assistive apparatus may include foot coupling portions 81 and 82 that are adjustably coupled in length or moveably or slideably coupled. The foot coupling portion 81 may be or include a heel fastening cap and a foot coupling portion 82 may be or include a foot side frame 82. The heel fastening cap and the foot side frame may be slideably coupled to each other.

The ankle joint assistive apparatus may include a rear frame 92 connected to a rear surface of the heel fastening cap 81, and an inter-shaft connecting frame 94 connecting the rear frame 92 and a shin coupling portion 95. The ankle joint assistive apparatus may include a first rotation shaft 91 arranged between the rear frame 92 and the inter-shaft frame 94, and a second rotation shaft 93 arranged between the inter-shaft frame 94 and the shin coupling portion 95. The first rotation shaft may rotate in a first direction, while the second rotation shaft 93 may rotate in a second direction.

The inter-shaft frame 94 may connect the first and second rotation shafts 91 and 93. A joint movement by the first and second rotation shafts 91 and 93 may be realized in the ankle joint assistive apparatus. However, since the first and second rotation shafts 91 and 93 are spaced apart from each other in the ankle joint assistive apparatus, points of action around which each rotation shaft 91 and 93 are rotated may be different from each other. An angle of the ankle may naturally be changed in three-dimensions at one point of action, but the ankle joint assistive apparatus structure rotating at multiple, different points of action does not perfectly correspond to a natural movement of the human body. As a result, the ankle joint assistive apparatus may impede or inconvenience an ankle movement of a wearer.

In addition, since each frame (i.e., a rear frame 92 or the inter-shaft frame 94) forming the ankle joint assistive apparatus may be formed of a metal material, an impact generated with the floor while walking may be transmitted to the user unchanged or unabsorbed. As described above, when the impact of the ankle joint assistive apparatus and the floor is transferred to the user unchanged, a user may easily tire and it may be difficult to achieve effective rehabilitation treatment due to a continuous impact.

Further, since the inter-shaft frame 94 of the ankle joint assistive apparatus may be arranged between the first and second rotation shafts 91 and 93, the ankle joint assisting apparatus may interfere with an ankle bone of the user during rotation. The foot ankle joint assistive apparatus may be uncomfortable due to a collision or interference between the ankle bone and the inter-shaft frame 94 during walking.

The above references are incorporated by reference herein where appropriate for appropriate teachings of additional or alternative details, features and/or technical background.

### BRIEF DESCRIPTION OF THE DRAWINGS

The embodiments will be described in detail with reference to the following drawings in which like reference numerals refer to like elements wherein:

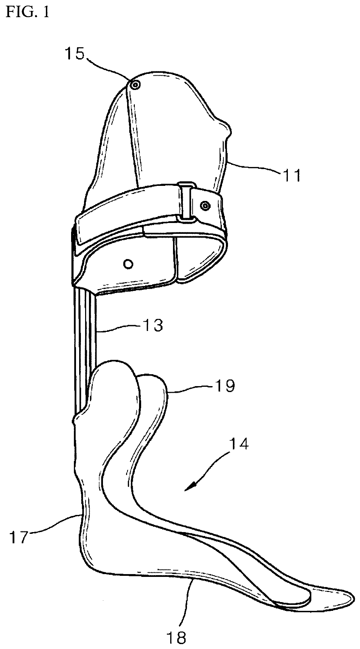

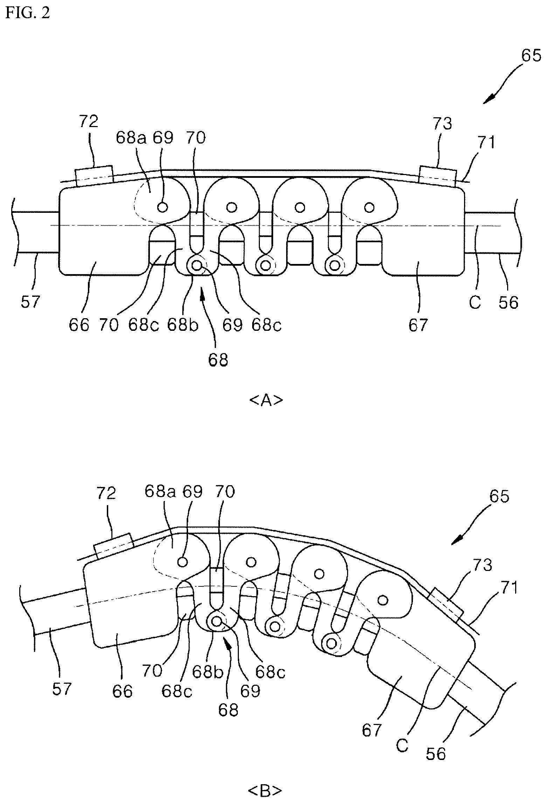

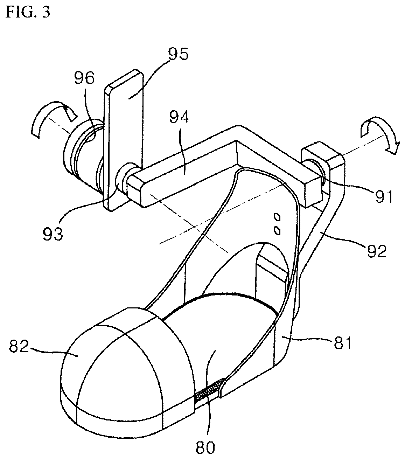

FIGS. 1 to 3 are views showing a conventional foot assisting apparatus or ankle assisting apparatus in accordance with prior art;
FIGs. 4A and 4B are perspective views showing a wearable assistive device such as an exoskeleton in accordance with an exemplary embodiment;
FIG. 5 is a side view showing the exoskeleton of FIG. 4;
FIG. 6 is a perspective view of a foot support of an exoskeleton in accordance with an exemplary embodiment of this application;
FIG. 7 is a side view showing the foot support of FIG. 6;
FIG. 8 is another side view showing the foot support of FIG. 6;
FIG. 9 is a top view showing the foot support of FIG. 6;
FIG. 10 is a rear view showing the foot support of FIG. 6;
FIG. 11 is a front view showing the foot support of FIG. 6;
FIG. 12 is an exploded perspective view showing the foot support of FIG. 6;
FIG. 13 is an exploded perspective view showing an ankle support of the foot of FIG. 12;
FIG. 14 is a perspective view showing the ankle support of FIG. 12;
FIG. 15 is a front view showing a body of the ankle support of FIG. 14;
FIG. 16 is a side view showing the body of the ankle support of FIG. 14;
FIG. 17 is a view illustrating a movement of the ankle support of FIG. 14;
FIGS. 18 and 19 are exploded perspective views of a connecting frame of FIG. 12;
FIG. 20 is a perspective view showing a foot to illustrate a connecting frame of FIG. 12;
FIG. 21 is a side view showing the foot support of FIG. 20;
FIG. 22 is a rear view showing the foot support of FIG. 20; and
FIG. 23 is a top view showing the foot support of FIG. 20.

### DETAILED DESCRIPTION

In the present specification, a term 'zigzag pattern' means a pattern in which one line or surface is repeated at a predetermined distance to form a bending. For example, 'the zigzag pattern' may be a shape in which the shapes such as a 'Z'-shape, a 'U'-shape, or a 'V'-shape are continuously arranged. The zigzag pattern may be an accordion pattern. An interval between a bending of the zigzag pattern may be formed all the same or may be formed different from each other. The zigzag pattern represents a structure capable of absorbing an impact applied from an outside.

Further, in the present specification, 'an assisting force' or 'assistive force' means an external force additionally provided to complement a user's natural motion or strength. The assistive force can be provided in a specific direction from an apparatus such as an electric motor or a hydraulic pump to generate the external force. The assistive force may move the exoskeleton in a direction that corresponds with a natural movement of the user. 'A bending' may correspond to bending in a first direction and extended to be curved or folded in a second direction different from the first direction.

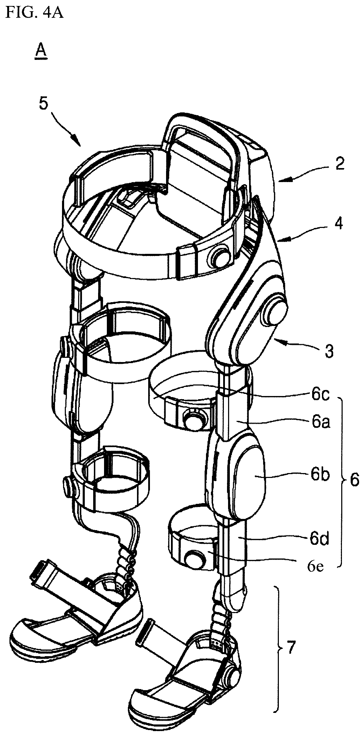

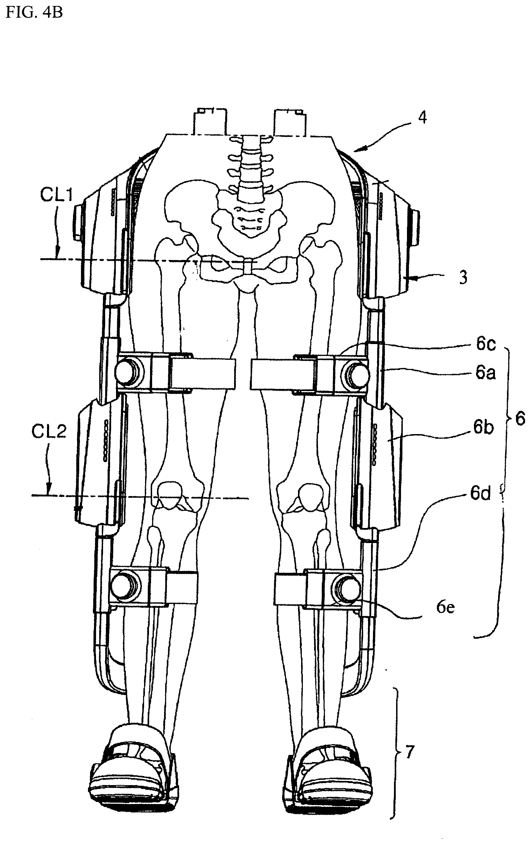

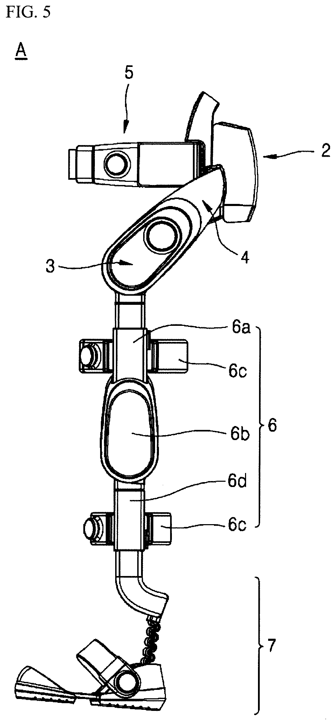

Referring to FIGS. 4A, 4B and 5, a wearable assistive device such as a wearable robot A, e.g., exoskeleton, may be worn on a lower body of a user, and may assist a lower body of the user by providing power or an assistive force during rehabilitation training such as walking.

Embodiments disclosed herein are not limited to an exoskeleton that fits onto a lower body of the user. For example, an exoskeleton A that fits onto an upper body of the user may be provided. Such an upper body exoskeleton A may include a pelvic frame and/or a lumbar frame, and a limb that couples to an arm of the user, for example. As another example, a full body exoskeleton or exoskeleton A that fits onto the upper body and the lower body of the user may also be provided.

The exoskeleton A may include a lumbar/back frame 2, an actuated hip joint 3, a main frame unit or main frame 4, and a subframe or pelvic/waist frame 5 that may be mounted on a pelvis and/or a waist of the user, a leg assembly or leg 6 that may be secured or coupled to a leg of the user, and a foot or shoe support 7 that may be coupled or secured to a foot or shoe of the user. The foot support 7 may alternatively be referred to as a foot assembly.

The lumbar/back frame 2 may be provided on the main frame 4 and may be provided at a rear side of the user when wearing the exoskeleton A. A main controller may be provided in the lumbar/back frame 2 and may adjust the width of the main frame 4 depending on a body size of the user. In addition, the lumbar/back frame 2 may include a battery pack that provides a power source to operate the exoskeleton A. The waist frame 5 may be provided on a first side, e.g., a front side, of the lumbar/back frame 2.

The waist/pelvic frame 5 may surround the user, and may support the waist. The waist/pelvic frame 5 may include a belt or strap that secures the waist of the user to the exoskeleton A. A length of the belt may be adjusted by a one-touch dial or knob. A portion of the waist/pelvic frame 5 that is in contact with the waist of the user may be made of a material capable of absorbing an impact to improve comfort.

The main frame 4 may support the back/lumbar frame 2 and may cover a first side, e.g., a left side, of a user's hip, and a second side, e.g., right side, of the user's hip. The main frame 4 may have a shape that surrounds or fits onto the user. The main frame 4 may be formed in an approximate 'U'-shape and may have first and second ends, and a bent portion or center section of the main frame 4 may be provided at the rear of the user. The lumbar/back frame 2 may be arranged on the center section of the main frame 4. However, this is merely one example, and a position of the lumbar/back frame 2 may be variously modified and implemented.

The first and second ends of the main frame 4 may be arranged downward along the pelvis, e.g., ilium, of the user when the user wears the exoskeleton A. Inclined portions or inclined extensions may be provided on first and second sides of the main frame 4, and the actuated hip joint 3 may be provided at a lower end of each inclined extension, respectively. The actuated hip joint 3 may include an actuator or a motor to provide the first assistive force in a rotational direction.

A subcontroller may be provided on or at the actuated hip joint 3 and may adjust a strength or magnitude of the first assistive force that assists the user. The subcontroller may adjust the first assistive force in a rotary dial manner. The actuated hip joint 3 or the inclined extension may be provided with an indicator to visually indicate the strength of the first assistive force. The indicator may include a lamp, light, or light-emitting device such as a light-emitting diode (LED). A driving means or motor providing the first assistive force may be encased in the actuated hip joint 3. The leg assembly 6 may be coupled to a lower side of the actuated hip joint 3.

Two leg assemblies 6 may be provided to assist both legs of the user. Each leg assembly 6 may include an upper leg frame 6a to be secured to a thigh of the user by a first leg strap or belt 6c, an actuated joint 6b housing an actuator or a motor to provide a second assistive force in a rotational direction, and a lower leg frame 6d to be secured to a calf of the user by a second leg strap or belt 6e. Lengths of the first and second leg belts 6c and 6e may be adjusted by using a one-touch dial. The actuated joint 6b may be provided between the upper leg frame 6a and the lower leg frame 6d.

The upper leg frame 6a, the actuated joint 6b, and the lower leg frame 6d may be arranged on an outer side of the leg of the user to allow a convenient bending movement of the user's joints during walking. The upper leg frame 6a may move based on the rotational actuation of the actuated hip joint 3 corresponding to a hip joint movement of the user. The lower leg frame 6d may move based on the rotational actuation of the actuated joint 6b corresponding to a knee joint movement of the user. The upper leg frame 6a and the lower leg frame 6d may thus move in forward and backward directions around axes CL1 and CL2 (FIG. 4B).

The main frame 4 may further have a hip joint structure (not shown) provided above the actuated hip joint 3. The hip joint structure in the main frame 4 may allow an outward movement, e.g., to a left side, of the end of the main frame 4 and the leg assembly 6. The user may therefore easily move his leg away from a midline of his body in a frontal plane of motion. In contrast, the actuated hip joint 3 may allow a forward and backward movement of the leg assembly 6 in a sagittal plane of motion.

The upper and lower leg frames 6a and 6d may also include a multi-frame structure and multi-joint structure. The multi-joint structure may allow outward and inward movement in the frontal plane, similar to a movement allowed by the hip joint structure in the main frame. The multi-frame structure may allow an adjustment in length via overlapping frames, while the multi-joint structure may allow an outward movement of the upper and lower leg frames 6a and 6d in the frontal plane.

The actuated joint 6b may generate the second assistive force, and may include a gear and motor set or actuator. For reference, the actuated joint 6b may be comprised of various driving means capable of generating the second assistive force.

The foot support 7 may be provided at a lower end of the lower leg frame 6d. The foot support 7 may support a barefoot of the user, a foot wearing socks, or shoes. For example, the exoskeleton A may include at least two leg assemblies 6, each having a foot support 7. Each foot support 7 may be coupled to a lower end of the leg assembly 6 and secure a shoe of the user to the exoskeleton A. The foot support 7 may have an adjustable length. A bottom section of the foot support 7 into which shoes are inserted may have slideably connected supports that accommodate various shoe sizes. The exemplary embodiment described may provide an example where the foot support 7 couples to a shoe of the user, but embodiments disclosed herein are not limited thereto.

The foot support 7 may have a fixing member, such as a strap, that secures an upper surface of the shoes to the foot support 7. The strap may be adjustable, so the foot support 7 can secure to various kinds, shapes, and sizes of shoes. The foot support 7 may be formed in a shape corresponding to the shoes of the user, and the fixing member may simply detach the shoes from the foot support 7. For example, the foot support 7 may have a sandal structure. However, a sandal structure of the foot support 7 is merely an example, and various shoes shapes and structures can be applied.

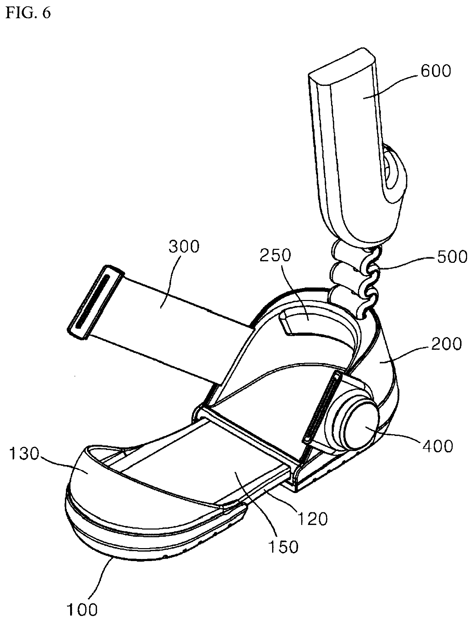

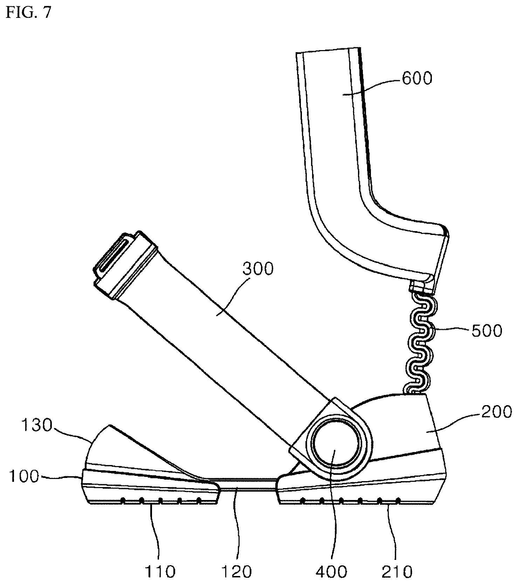

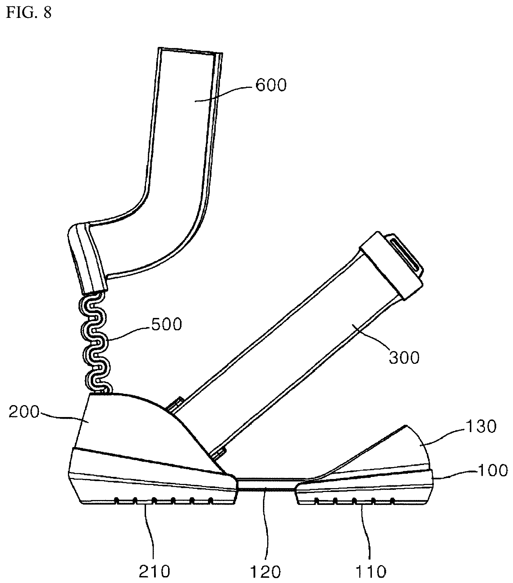

Referring to FIGS. 6 to 12, the foot support 7 in accordance with an exemplary embodiment may include first, e.g., front, and second, e.g., rear, supports 100 and 200, a binding belt or strap 300, a button dial or knob 400, an ankle support or ankle assembly 500, and a connecting frame portion or connection frame 600. The ankle assembly 500 may include a flexible member.

The first and second supports 100 and 200 may partially cover the user's shoes. For example, the first and second supports 100 and 200 may cover the toe and heel portions of the shoe, and may be slideably coupled to each other. The first and second support 100 and 200 may together comprise a foot supporting structure.

As another example, the first and second supports 100 and 200 may be shaped to fit the shoe of the user, and may include a structure that simply detaches from the shoes such as a sandal structure or strap. Any method that secures the shoes of the user to the foot support 7 can be applied to the shape and/or structure of the first and second supports 100 and 200.

The first support 100 may have a structure to support a front of the shoes. The first support 100 may cover a portion of the front of the shoes near or over the toes. The first support 100 may have a curved semi-spherical or semi-ellipsoid shape. The first support 100 may be rounded to surround a front section of the shoe of the user.

The first support 100 may include a first outer sole 110 that touches to a floor surface or a ground, a midsole or a shank 120, and a front support or toe cover 130 extending upward at a first end of the midsole 120. The first outer sole 110 may grip the surface of the floor and increase a friction between the foot support 7 and the floor. The midsole 120 may include a section that is inserted into the second support 200 such that the first support 100 and the second support 200 are slideably connected. The second support 200 may also have an inner sole (not shown). The toe cover 130 may extend upward and may have a curved semi-spherical or semi-ellipsoid shape that partially covers toes or a front portion of the shoe of the user. The toe cover 130 may also be referred to as a toe box or toe cap.

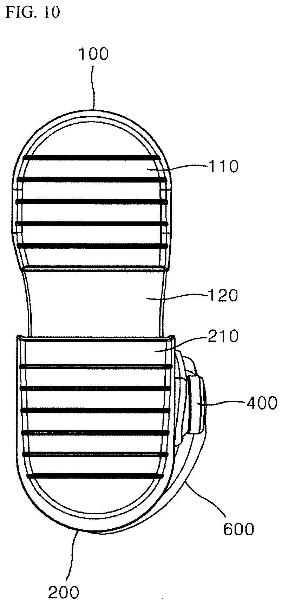

The first outer sole 110 may include a variety of patterns formed on a bottom surface that touches the ground and that is capable of increasing a frictional force with the ground. Although FIG. 10 shows a linear pattern on the first outer sole 110, this application is not limited thereto, and the first outer sole 110 may be formed with various patterns such as a diagonal line, a zigzag pattern, a concave-convex surface, etc.

The midsole 120 may be provided above the first outer sole 110. The midsole 120 may have a long and flat shape to support a front of the shoes of the user. The midsole 120 may be partially inserted into an inner space of the second support 200, and the user may slide the midsole 120 in and out of the second support 200 to adjust the length of the foot support 7. An entire length of the foot support 7 may be adjusted depending on a length the midsole 120 that is inserted into the inner space of the second support 200. When the foot support 7 is lengthened by withdrawing the midsole 120 from the second support 200, a portion of the midsole 120 may spaced apart from the ground.

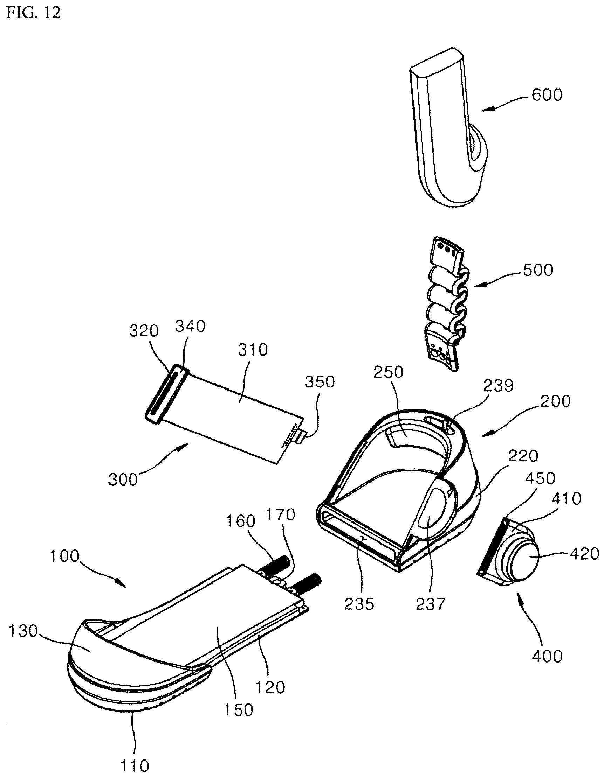

As an example, referring to FIG. 12, the second support 200 may include a receiving space or inner space 235 in which an elastic member 160 is installed and a wire roller 170, which may extend out from the midsole 120. The midsole 120 may further include an upper surface 150. The upper surface 150 include a layer made of a soft, elastic, or shock absorbing material to provide comfort to the user and to absorb any impact to serve as an inner sole.

The toe cover 130 may have a shape that partially covers a front of the shoes to restrict a movement of the toes and to prevent the foot support 7 from accidently slipping or falling off. The toe cover 130 may smoothly protrude inward toward the midsole 120 so as to form a curved surface upward from the first end of the midsole 120. The toe cover 130 may form an acute angle with the midsole 120, thereby partially covering from front of the shoes while exposing most of an upper surface of the shoes.

The second support 200 may have a shape that supports a rear of the shoes. The second support 200 may have an inner space or receiving portion in which the midsole 120 of the first support 100 may be inserted. The receiving portion may be provided at an interior of the second support 200. As previously described, a part of the first support 100 may be inserted into the second support 200 such that it may be slidingly or slideably moved, adjusting a length between the first support 100 and the second support 200.

A lower surface of the second support 200 may be formed with a second outer sole 210, which may be provided with various patterns configured to grip with the surface of the floor and/or increase a frictional force with the ground. Like the first outer sole 110, a linear pattern is shown on the second outer sole 210 in FIG. 10. However, embodiments are not limited thereto, and various patterns such as a diagonal line, a zigzag pattern, and various concave recesses and/or convex protrusions etc., may be formed on the second outer sole 210.

A heel cover or heel cushion 250 may be arranged on an inner side of the second support 200 so that the rear of the shoes may be restricted by the second support 200. The second support 200 may extend upward and may have a curved semi-spherical or semi-ellipsoid shape that partially covers a heel or back portion of the shoe of the user. The heel cushion 250 may be provided on an inner side of the portion of the second support 200 that extends upward.

Alternatively, the first support 100 and the second support 200 may be integrally formed. The first support 100 and the second support 200 may support a lower surface of the shoes of the user as an integral structure and may not be slideably adjusted. For example, the first and second supports 100 and 200 may be formed in a flat panel structure to support the shoes. However, this is merely one example, and the first and second supports 100 and 200 may be formed in various shapes that can support the shoes.

As another example, the first and second supports 100 and 200 may be integrally formed with the ankle support 500. The first and second supports 100 and 200 may be made of the same material as the ankle support 500, such as a single elastic material. However, this is merely any one of various embodiments that can be changed, and disclosed embodiments are not limited thereto.

The strap 300 may extend from a first side of the second support 200, and may couple to a second side of the second support 200. The strap 300 may run literally across the user's shoes. The strap 300 may be coupled with the knob 400 and may secure the shoe of the user to the foot support 7 of the exoskeleton A.

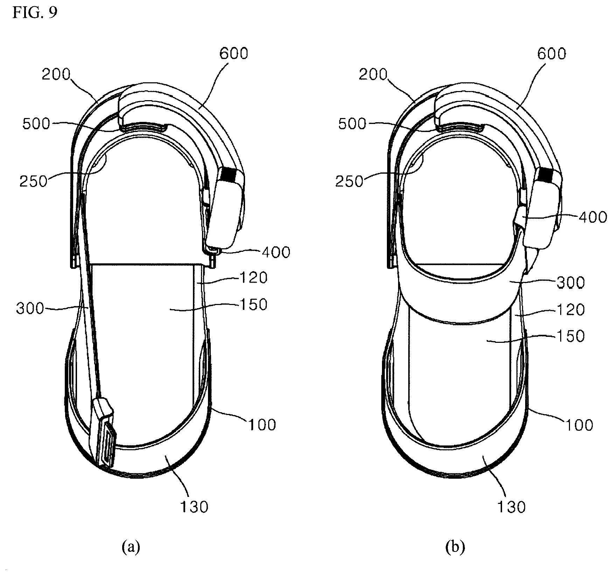

Referring to FIG. 9, FIG. 9(a) shows the foot support 7 before fastening the strap 300 to the knob 400, and FIG. 9(b) shows the foot support 7 after fastening the strap 300 and the knob 400. The strap 300 may be inserted partially into the knob 400, and a length thereof may be adjusted in accordance with the size or width of the shoes of the user. As a result, the strap 300 may prevent the foot support 7 from accidentally falling off the user's shoes.

The knob 400 may be provided on the second side of the second support 200 and may be coupled with the strap 300 to adjust a length of the strap 300 exposed to an outside of the foot support 7. The knob 400 may include a combination of internal parts and may have a rotatable dial protruding from a side of the second support 200.

The knob 400 may fasten, adjust and/or separate the strap 300 to from the knob 400 via a one-touch dial method. For example, when a dial of the knob 400 is rotated in a state in which the strap 300 is coupled to the knob 400, the length of the strap 300 may be adjusted to change a tension or tightness of the strap 300. Further, when the dial of the knob 400 is pulled outward, the strap 300 fastened to the knob 400 may be separated from the knob 400. However, this is merely one example, and the knob 400 can be operated in various ways.

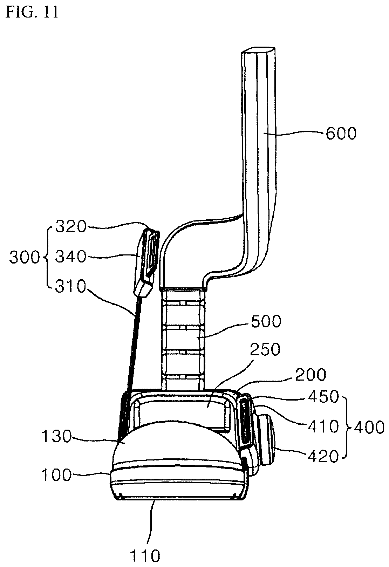

Referring to FIG. 11, as another example, the strap 300 may include a band 310, which may be flexible, a binding clip cover or buckle 340, which may be rigid, and a tongue or latch 320 provided on the buckle 340. The tongue 320 may also be referred to as a binder clip, clasp, or protuberance. The tongue 320 may couple to the knob 400. The knob 400 may include a button or dial 420, a coupling member or housing 410, and a receiving space 450 in which the tongue 320 of the strap 300 may be inserted. The receiving space 450 may be provided in the housing 410 of the knob 400. In this example, the button 420 may be pressed to release the tongue 320 from the receiving space 450.

Referring to FIG. 12, the strap 300 may include a wire guide 350 at an opposite end from the tongue 320 which may installed in the second support 200. An optional ledge or coupling groove 237 may be formed to extend from the second side of the second support 200.

The ankle support 500 may partially cover an Achilles tendon of the user who wears the foot support 7. A first end of the ankle support 500 may be fixed at an upper portion or top of the second support 200. The ankle support 500 may be centrally positioned at the second support 200. Further, a second end of the ankle support 500 may be fixed to the connection frame 600.

The ankle support 500 may have a winding or bending shape having a predetermined width. For example, the ankle support 500 may have a cross-section where a ' 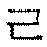 '-shape is repeated. The cross-section may be a zigzag or accordion shape. The ankle support 500 may be formed to have various cross sections and widths. Due to these shapes, the ankle support 500 may be stretched and contracted within a predetermined length range.

Accordingly, the ankle support 500 may allow the foot support 7 to have a free range of rotation in all directions (e.g., forward, rearward, leftward, rightward, upward, and downward) based on the connection frame 600 and depending on an ankle movement of the user. The ankle support 500 may therefore expand a moveable range of the foot support 7 or allow a free range of motion of the shoe of the user. The ankle support 500 may be moved at one point of action (or one point of rotation) in the ankle support 500. As a result, the ankle support 500 can support the ankle so as to allow a natural and/or complex ankle movement of the user. A structure of the ankle support 500 may allow the user to easily change directions as desired, thereby increasing a degree of freedom of a movement of the user.

The ankle support 500 may minimize an unnaturalness or discomfort that the user could feel while wearing the exoskeleton A during walking. Although an impact with the ground may be increased in proportion to a weight of the user and the exoskeleton A, the ankle support 500 may mitigate this impact, minimizing fatigue and unsteadiness and maximizing the user's control over his body.

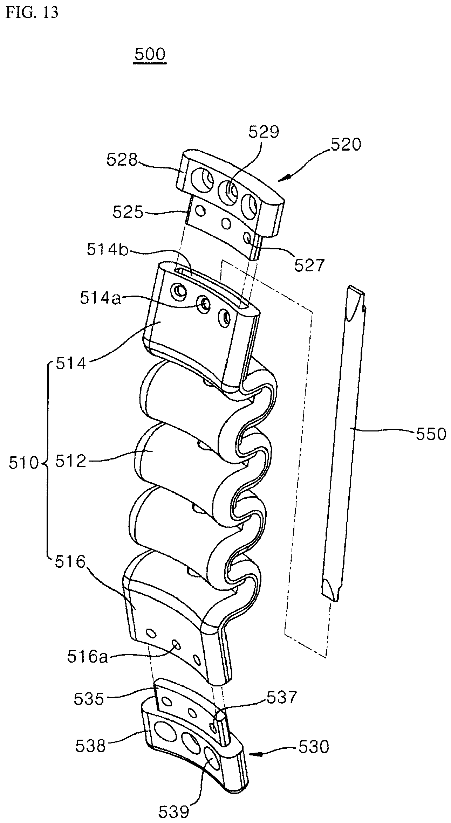

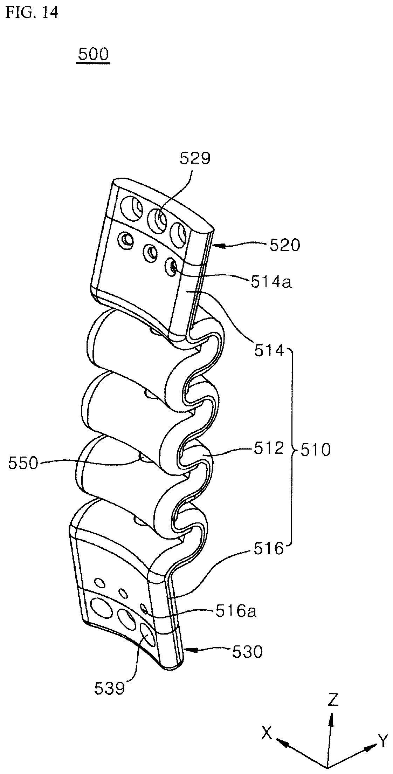

Referring to FIGS. 13 to 16, the ankle support 500 may include a body module or ankle body 510, a first holder or frame 520, a second holder or frame 530, and a stabilizer shaft 550 described later. The ankle body 510 may include an Achilles extension 512 having an accordion spring or accordion boot. The Achilles extension may also be referred to as a pattern member formed with a pattern having repeated bends or folds. The ankle body 510 may further include first and second connectors or connection ends 514 and 516 extending top and bottom ends of the Achilles extension 512, respectively. The top and bottom ends of the Achilles extension 512 may also be referred to as first and second ends, respectively. The first and second connectors 514 will be described later.

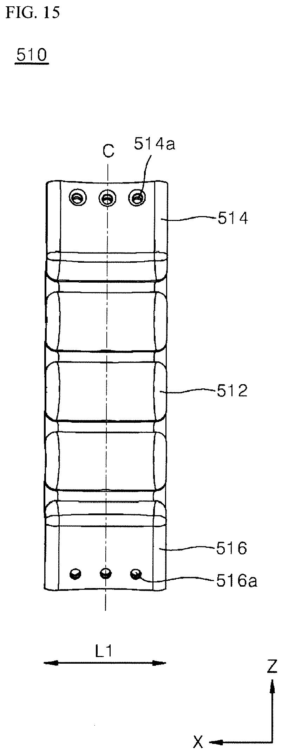

A cross-section of the Achilles extension 512 may appear to be an accordion or zig-zag. A material of the Achilles extension 512 may be repeatedly bent or folded in, for example, a zigzag pattern, an accordion pattern, a ' 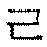 '-shaped pattern, or a ' 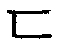 '-shaped pattern. The pattern which forms the Achilles extension 512 may allow expansion and contraction. The Achilles extension 512 may have a constant width (L1 in FIG. 15) in a first direction (e.g., an X-axis direction). As an example, the width of the Achilles extension 512 may be about 25-45 mm.

Alternatively, the Achilles extension 512 may be formed such that an upper width of the Achilles extension 512 and a lower width of the Achilles extension 512 are smaller than a center width. The width of the Achilles extension 512 may be variously modified and implemented.

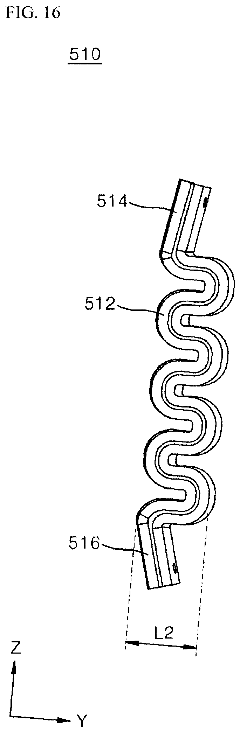

The bends and folds of the Achilles extension 512 may vertically align or overlap on top of each other in a longitudinal direction (e.g., a z-axis direction). For example, the Achilles extension 512 may be formed in a zigzag pattern having about seven bends or folds. The Achilles extension 512 may include more folds to increase a height of the Achilles extension 512. A length (L2 in FIG. 16) of the Achilles extension 512 measured in a second direction (e.g., a Y-axis direction) may be about 25 to 30 mm. However, embodiments disclosed herein are not limited thereto, and the number and shape of the bends can be variously modified and implemented.

A shape of the Achilles extension 512 may act as a spring to absorb an external impact. Further, the Achilles extension 512 may be made of an elastic material to more effectively absorb the external impact.

The elastic material forming the Achilles extension 512 may be formed of a natural rubber, a nitrile rubber, a synthetic rubber, a silicone rubber, ethylene propylene diene monomer rubber (EPDM), polybutadiene, or polyurethane, for example. Such an elastic material can be produced by a wet spinning, a dry spinning, a melt spinning, and/or a polymerization reaction spinning method, etc. The elastic material may also be produced by mixing in a fabric or fiber having an elasticity or stretch. However, this is merely one example, and the kinds and a manufacturing method of the elastic material forming the Achilles extension 512 are not limited thereto. These methods and materials may also be used to form the elastic material in an embodiment where the first support 100, second support 200, and the ankle support 500 are integrally formed of the elastic material. The Achilles extension 512 may absorb an impact that can be generated while wearing the exoskeleton A while walking.

The shape and material of the Achilles extension 512 may allow the Achilles extension 512 to freely rotate (e.g., upward, downward, forward, rearward, leftward, and/or rightward) to expand a moveable range of the foot support 7. The Achilles extension 512 may rotate in a three-dimensional angle relative to the connection frame 600 to support the user's ankle in accordance with a complex ankle movement, and can therefore provide a high degree of freedom. For example, the Achilles extension 512 may allow the foot support 7 of the exoskeleton A and/or the foot of the user to rotate in a transverse plane of the user, may allow rotation such as plantar flexion and dorsiflexion in the sagittal plane of the user, or may allow a pivot motion such as ankle eversion and ankle inversion in the frontal plane of the user.

The first connector 514 may extend in the longitudinal direction (i.e., the Z-axis direction) from the top end of the Achilles extension 512. A first connection slot or opening 514b and at least one first connection hole 514a may be provided on a top side of the first connector 514, and may be coupled to a first holder or frame 520 having a first bracket 528 and first protuberance 525.

One or more first connection holes 514a may have a circular or polygonal shape on the top side of the first connector 514. When a plurality of first connection holes 514a are formed in the first connector 514, a plurality of first connection holes 514a may be arranged at an equal interval to disperse an applied force. The first connection hole 514a may penetrate a surface of the first connector 514 so that a coupling member or a fastener such as a bolt may pass through the first connection hole 514a.

At least one first connection hole 514a may communicate with or intersect a space of the first connection slot 514b formed inside the first connector 514. The first connection slot 514b may be a concave recess or a hollow inner surface of the first connector 514. The first connection slot 514b may be formed to have a smaller size than that of the first connector 514. Although one first connection slot 514b is shown on an end surface of the first connecting member 514 in the figures, a plurality of first connection slots 514b, may be arranged on the end surface of the first connector 514. In this alternative embodiment, the first connection slots 514b may be smaller so that they fit on the end surface of the first connector 514.

The first protuberance 525 extending from the first bracket 528 may be inserted into the first connection slot 514b. The first protuberance 525 may have a shape corresponding to the first connection slot 514b and may have a size equal to or smaller than a size of the first connection slot 514b. At least one second connection hole 527 formed in the first protuberance 525 may overlap with at least one first connection hole 514a when the first protuberance 525 is inserted into the first connection slot 514b. At least one third connection hole 529 may be provided on the first bracket 528. The size of the first bracket 528 may be greater than that of the first protuberance 525. The third connection hole 529 may secure the Achilles extension 512 to the connection frame 600 and will be described later.

Although not explicitly shown, the coupling between the first connector 514 and the first protuberance 525 may be fixed or secured by a coupling member or fastener, such as a bolt penetrating the first and second connection holes 514a and 527 together. The coupling member is not limited to a bolt and may have various configurations such as a screw, an adhesive filler, or a fixing clip.

Like the first connector 514, the second connector 516 may extend in the longitudinal direction (the Z-axis direction) from the bottom end of the Achilles extension 512. A second connection slot (not shown) and at least one fourth connection hole 516a, may be formed on the second connector 516.

At least one fourth connection hole 516a may be formed on a lower side of the second connector 516. Since the fourth connection hole 516a may be substantially the same as the above described first connection hole 514a, a repeated description will be omitted.

At least one fourth connection hole 516a may be formed to intersect or communicate with a space of the second connection slot formed inside the second connector 516. The second connection slot may be formed substantially the same as the above described first connection slot 514b, so a repeated description will be omitted.

A second holder or frame 530 may include a second bracket 538 and a second protuberance 535. The second protuberance 535 may be inserted into the second connection slot of the second connector 516. At least one fifth connection hole 537 formed in the second protuberance 535 may overlap with the fourth connection hole 516a when the second protuberance 535 is inserted into the second connection slot of the second connector 516. A coupling of the second connector 516 and the second protuberance 535 may be substantially the same as a coupling between the first protuberance 525 and the first connector 516, and may involve a bolt that penetrates through the fourth and fifth connection holes 516a and 537. The second bracket 538 may at least one sixth connection hole 539 to couple to the second support 200 and will be described later.

The Achilles extension 512, the first connector 514, and the second connector 516 may be formed together integrally. Further, the Achilles extension 512, the first connector 514, and the second connector 516 may be made of the same material. For example, the Achilles extension 512, the first connector 514, and the second connector 516 may all be made of the above-described elastic material.

At least one second connection hole 527 that couples with or connects to the first connector 514 may be formed on the first protuberance 525. The second connection hole 527 may have a shape and a size corresponding to the first connection hole 514a formed on the first connector 514. The number of second connection holes 527 may be the same as the number of first connection holes 514a, and at least one second connection hole 527 may further have a position aligning with a position of at least one first connection hole 514a.

The first bracket 528 may have the same width and thickness as that of the Achilles extension 512. At least one third connection hole 529 provided on the first bracket 528 may be used to couple with the connection frame 600. The third connection holes 529 may have in a circular or polygonal shape. When a plurality of third connection holes 529 are formed on the first bracket 528, the plurality of third connection holes 529 may be spaced apart by equal intervals to disperse any applied force.

A coupling member or a fastener such as a bolt may pass through the third connection hole 529 so that the second bracket 538 may be coupled to a housing coupling portion or coupler 625 of the connection frame 600 to be described later. However, this is merely one example, and the third connection hole 529 may be formed in various shapes so that various coupling members or coupling methods may be applied.

The first holder 520 may connect the ankle support 500 and the connection frame 600. The first holder 520 may be formed of a different material than a material of the ankle body 510. For example, the first holder 520 may be formed of a more rigid material than the ankle body 510. The first holder 520 may be formed of a material including a plastic or a metal material, or may be formed of various inelastic materials. The second holder 530 may have substantially the same structure and shape as the first holder 520.

The second holder 530 may include the second protuberance 535 and the second bracket 538. The second protuberance 535 may be smaller than the second bracket 538 and may be inserted into the second connection slot of the second connector 516. Accordingly, the second protuberance 535 may have a size and a shape corresponding to the second connection slot. The size of the second protuberance 535 may be equal to or smaller than the size of the second connection slot.

The second protuberance 535 may include at least one fifth connection hole 537 that couples with the second connector 516. At least one fifth connection hole 537 may have a shape and a size corresponding to that of the fourth connection hole 516a formed on the second connector 516. The number of fifth connection holes 537 may be the same as the number of fourth connection holes 516a, and the fifth connection hole 537 may have a position aligning with a position of the fourth connection hole 516a. A coupling member or a fastener, e.g., bolt, screw, shaft, etc., may be inserted through the fifth connection hole 537 and the fourth connection hole 516a to couple the third holder 530 to the second connector 516, but embodiments are not limited thereto.

The second bracket 538 may have a width and thickness equal to a width and thickness of the Achilles extension 512. At least one sixth connection hole 539 may be formed on the second bracket 538 to couple with the second support 200 of the foot support 7.

The second holder 530 may be inserted into a rear slot or rear recess 239 (see FIG. 12), which may be formed at a center of the rear surface of the second support 200. The second support 200 may include holes on the rear recess 239, and a bolt may secure the second support 200 to the second bracket 538. The heel cushion 250 may be placed over the bolts in the second support 200 to cover the bolts. The second support 200 and/or the rear recess 239 may include an alternative fastener that secures to the sixth connection holes 539, and embodiments disclosed herein are not limited to bolts or shafts. Alternatively, the second holder 530 may be formed integrally with a rear surface of the second support 200.

The second holder 530 may be formed of a different material from the ankle body 510. The second holder 530 may connect the ankle support 500 and the second support 200 and may be formed of a more rigid material than that of the ankle body 510. The second holder 530 may be formed of the same material as the first holder 520. The first and second holders 520 and 530 may be made of metal, for example. The first and second connectors 514 and 516 may also be made of metal.

The stabilizer shaft 550 may penetrate the Achilles extension 512 in the longitudinal direction (the Z-axis direction) so that a part of the stabilizer shaft 550 may be exposed in spaces between the folds of the Achilles extension 512. In other words, the stabilizer shaft 550 may be threaded through the repeated bends and folds of the Achilles extension 512 such that the stabilizer shaft 550 intersects the folds.

The stabilizer shaft 550 may have a different color from the Achilles extension 512, and a color contrast between the stabilizer shaft 550 and the Achilles extension 512 may enhance an aestheticism of the foot support 7. The stabilizer shaft 550 may be arranged on a center line (C in FIG. 15) of the ankle body 510, or through a center of the folds of the Achilles extension 512. However, this is merely one example, and there may be two stabilizer shafts 550 arranged on both sides of the ankle body 510 based on the center line (C in FIG. 15). An arrangement of the stabilizer shaft 550 may be variously modified and implemented.

The stabilizer shaft 550 may be a hollow cable or wire cover that holds an optional wire or data cable. Alternatively, the stabilizer shaft 550 may be a solid shaft or extension that is not hollow. The stabilizer shaft 550 may be flexible so as not to restrict a movement of the Achilles extension 512. The stabilizer shaft 550 may be more rigid than the Achilles extension 512 to provide stability to the Achilles extension 512. Alternatively, the Achilles extension 512 may be rigid enough to support itself, and the stabilizer shaft 550 may be optional.

Although not explicitly shown in the drawings, a wire inside of the rod 550 may be electrically connected to a first pressure sensor (not shown) in the first support 100 and a second pressure sensor (not shown) in the second support 200. The wire in the rod 550 may be formed of a data cable to transmit a signal generated in the first pressure sensor and the second pressure sensor.

Although not explicitly shown in the drawings, the stabilizer shaft 550 and/or the wire in the stabilizer shaft 550 may extend upward along an inside of the leg assembly 6. The wire in the stabilizer shaft 550 may transmit a signal generated in the first pressure sensor and at the second pressure sensor to a main controller and/or a subcontroller provided at the actuated hip joint 3. The main controller or the subcontroller may calculate a change in a center of gravity of the user by using, for example, a signal transmitted through the wire from the first and second pressure sensors and a magnitude and direction of the first and second assistive forces.

An arrangement of the wire in the stabilizer shaft 550 penetrating the Achilles extension 512 in the longitudinal direction (the Z-axis direction) may prevent the wire from being caught or twisted by an external object. Further, a production cost of the exoskeleton A may be reduced by minimizing an amount of wire required for a signal transmission. The stabilizer shaft 550 penetrating the Achilles extension 512 may improve an aestheticism of the foot support 7.

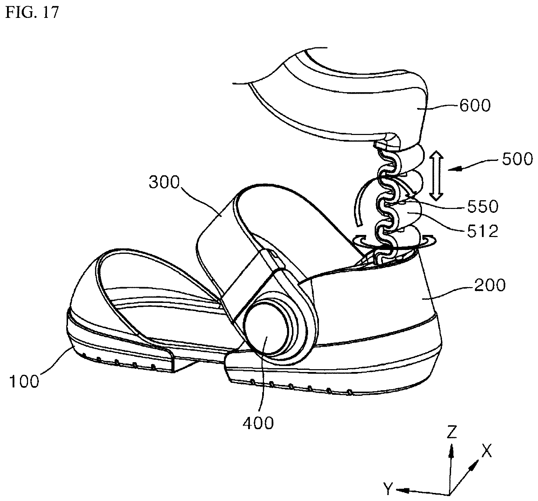

Referring to FIG. 17, the ankle support 500 may couple the second support 200 to the connection frame 600. The configuration of the ankle support 500 and/or the Achilles extension 512 may allow the foot support 7 to move and rotate freely in the x, y, and z-axis directions relative to the connection frame 600 of the leg assembly 6. The elasticity of the ankle body 510 and/or the Achilles extension 512 may also absorb a shock or impact from the ground as the user walks. The user may, for example, easily rotate their feet in a transverse plane, perform plantar flexion and/or dorsiflexion in a sagittal plane, or perform ankle inversion or eversion in a transverse plane. The user may easily rotate their toes or heels, and may easily walk while wearing the exoskeleton A. In an alternative embodiment, the connection frame 600 may be omitted. In this case, the ankle support 500 may be directly connected to the lower end of the leg assembly 6.

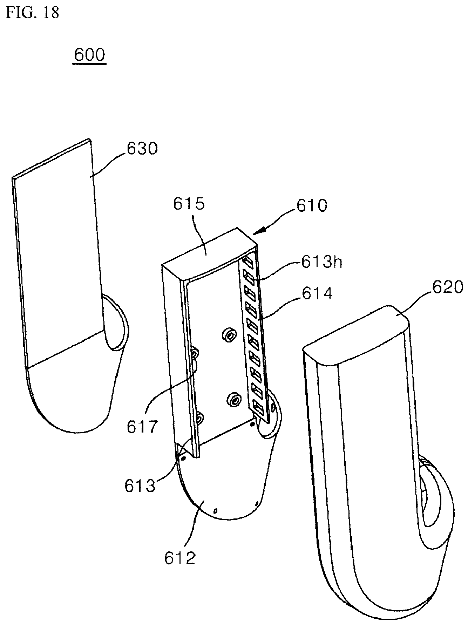

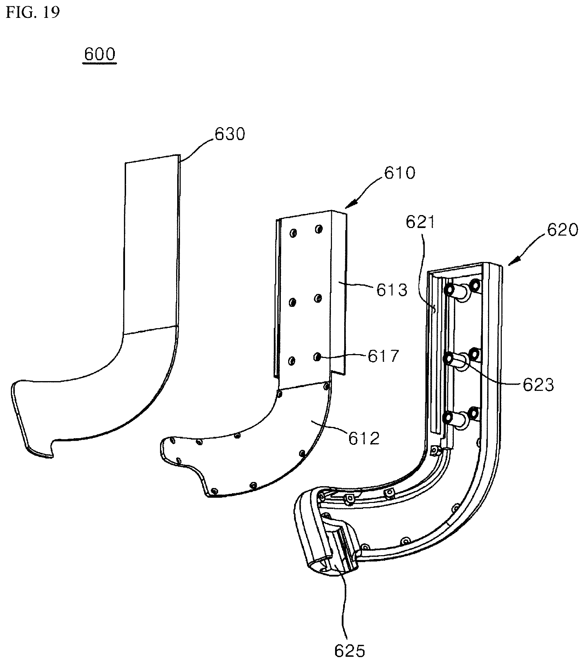

Referring to FIGS. 18 and 19, the connection frame 600 may include a skeleton frame 610, a frame housing 620, and a frame cover 630. The connection frame 600 may extend along a side of the leg of the user and may bend to extend downward toward a rear of the shoes of the user so as not to interfere with an ankle bone or malleolus of the user during walking. The connection frame 600 may have a curved shape such it couples the ankle support 500 (provided at a rear of the foot) to the leg assembly 6 (provided on a side of a leg of the user). The connection frame 600 may begin to bend above the lateral malleolus so as not to overlap with the ankle.

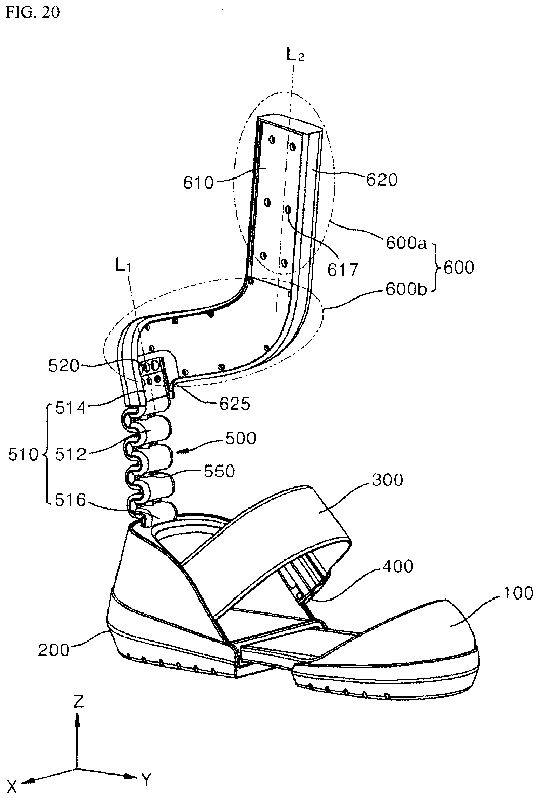

A first portion or rear support (600b in FIG. 20) of the connection frame 600 may extend in a longitudinal direction from the ankle support 500 behind the Achilles tendon, and a second portion or side support (600a in FIG. 20) of the connection frame 600 may extend to cover an outer side of the leg of the user. As a result, the connection frame 600 may minimize an interference between a user's joint (for example, the ankle joint proper, the subtalar joint, and/or the inferior tibiofibular joint) and the exoskeleton A during walking. The connection frame 600 may minimize physical impact between the leg of the user and the exoskeleton A during walking and help control the body of the user. The user may be able to use the exoskeleton A for a relatively long time due to increased comfort and convenience, improving rehabilitation.

The side support (600a in FIG. 20) may extend along a side of a leg of a user and the rear support (600b in FIG. 20) may bend from the side support 600a and extend to a rear of the leg of the user. The rear support 600b may also be referred to as a bending portion or extension.

The skeleton frame 610 may be arranged inside the connection frame 600 and may form a center skeleton or frame of the connection frame 600. The skeleton frame 610 may have a shape corresponding to the side or rear supports 600a and 600b. In other words, the skeleton frame 610 may have a cross-sectional shape that corresponds to the general shape of the connection frame 600.

Referring to FIG. 18, the skeleton frame 610 may include a base plate 612 that forms a base shape of the side and rear supports 600a and 600b, and a first plate or first wall 613 formed on a first side of the base plate 612. Further, the skeleton frame 610 may include a second plate or second wall 614 formed on a second side of the base plate 612, and a third plate or a third wall 615 connected to an end of the first and second walls 613 and 614 and the base plate 612. The first to third walls 613, 614, and 615 may be formed on an upper side or top portion of the base plate 612.

The base plate 612 and the first and second walls 613 and 614 may together be arranged to have a ' 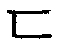 '-shaped cross-section. The third wall 615 may support the first and second walls 613 and 614 and may be fixed on the base plate 612. Due to this structure, the skeleton frame 610 may not be easily warped or transformed in shape by an external force.

The first to third walls 613, 614 and 615 may be arranged on an area corresponding to the side support 600a of the connection frame 600. An arrangement of the first to third side wall plates 613, 614, and 615 may be modified variously and implemented. For example, a plurality of length adjusting grooves or notches 613h may be arranged on the second wall 614. There may also be a plurality of notches 613h on the first wall 613. In an alternative embodiment, the notches 613h may be holes or openings.

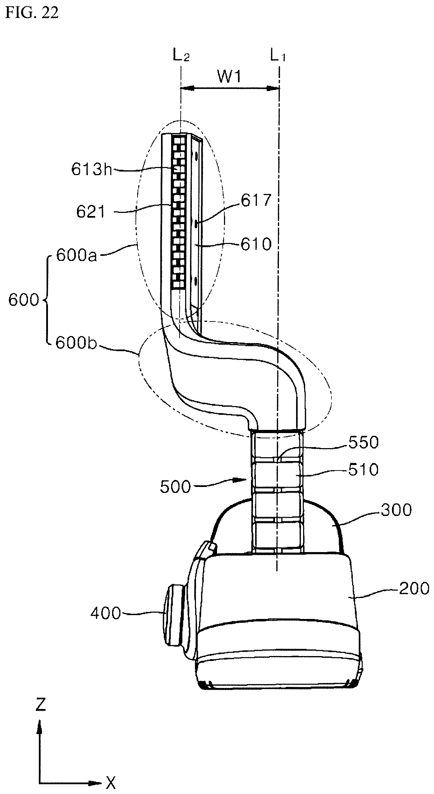

The plurality notches 613h which may be exposed to or correspond to a length adjusting portion or opening 621 provided on the frame housing 620. The notches 613h may form grooves or recesses, may protrude, or may be a series of alternating indentations and protrusions. For example, FIG. 18 shows an example where the notches 613h may appear to be recesses or grooves on an inner side of the second wall 614, and may be formed to protrude on an outer side of the second wall 614. FIG. 22 shows an example where the notches 613h may appear to be alternating outward notches and inward recesses or holes. The notches 613h may have a shape that is configured to couple with corresponding protrusions or notches (not shown) of the lower leg frame 6d. In an alternative embodiment where the notches 613h are holes or openings, protrusions or pins of the lower leg frame 6d may be inserted or screwed into the holes 613h.

The notches 613h may protrude into or be exposed by the opening 621 to couple to corresponding protrusions provided on an end of the lower leg frame 6d so that the connection frame 600 can couple to the rest of the leg assembly 6. The coupling between the notches 613h and the protrusions through the opening 621 may be used to adjust the length of the connection frame 600 at a calf portion of the leg assembly 6. The size, spacing, and number of the notches 613h may be variously modified. Alternatively, the notches 613h may be formed on a separate member that is coupled onto the first wall 613 and second wall 614.

A calf length of the exoskeleton A may thus be adjusted by an overlapping length of the connection frame 600 with an end of the lower leg frame 6d that has the protrusions. In an alternative embodiment, the notches 613h may instead be pins or shafts that are inserted into holes or recesses of the lower leg frame 6d. As another alternative embodiment, the notches 613h may instead be recesses or holes into which pins or shafts of the lower leg frame 6d may be inserted. A coupling of the connection frame 600 and the lower leg frame 6d will be described in further detail later. The coupling may be secured by a press-fitting of the protrusions or notches of the lower leg frame 6d into the notches 613h, or by screwing pins or shafts into corresponding holes. However, embodiments disclosed herein are not limited to the above-described coupling methods.

Referring to FIG. 19, a portion of the base plate 612 corresponding to the rear support 600b may cover or wrap around a circumference of the leg of the user. The base plate 612 may be curved in a direction opposite a direction in which the first through third walls 613, 614, and 615 extend.

A plurality of plate fasteners or fastener holes 617 formed on the base plate 612 may couple to fasteners or pins 623 of the frame housing 620. Alternatively, the fastener holes 617 may be grooves. The shape, number, and position of the fastener holes 617 may be variously modified as needed and implemented. The fastener holes 617 may further couple to fasteners or pins (not shown) of the frame cover 630. The pins or fasteners of the frame cover 630 may be similar to the pins 623 of the frame housing 620, and may face the pins 623. A plurality of fastener holes may also be formed on a bottom portion of the base plate 612, which may couple to fastener holes of the frame housing 620 via screws or bolts, for example. However, a coupling method of the skeleton frame 610, frame housing 620, and frame cover 630 is not limited thereto. For example, the frame cover 630 and the skeleton frame 610 may be press fit into the frame housing 620.

The skeleton frame 610 may be formed of a rigid metal material, but is not limited to metal. The skeleton frame 610 may instead be formed of other various rigid materials. In an alternative embodiment, the base plate 612 and the first to third walls 613, 614 and 615 may be integrally formed in one process.

The frame housing 620 may have a shape covering a first surface of the skeleton frame 610. The first to third walls 613, 614 and 615 may be inserted into the frame housing 620. The frame housing 620 may have a coupling groove that is formed or recessed concavely inward to couple with the first to third walls 613, 614, and 615.

The frame housing 620 may have a substantially similar curved shape as that of the base plate 612 and the skeleton frame 610. On an inner surface of the frame housing 620, at least one fastener or pin 623 corresponding to at least one fastener hole 617 of the skeleton frame 610 may be formed. The pin 623 may have a shape, number, and position corresponding to those of at least one fastener hole 617.

The opening 621 may be formed at a first side of the frame housing 620. There may also be an opening 621 on a second side of the frame housing 620. The plurality of notches 613h may adjust a lower calf length of the exoskeleton A created by the lower leg frame 6d and the connection frame 600. The notches 613h of the skeleton frame 610 may be exposed to an outside of the connection frame 600 through the opening 621, and may be configured to couple to protrusions provided on the lower leg frame 6d.

For example, the lower leg frame 6d may have a pair of extensions that are spaced apart by a length equal to a length of the third wall 615 such that a bottom portion of the lower leg frame 6d may have a n-shape. There may be a receiving space between the two extensions, and at least one of the extensions may have protrusions on inner sides such that the protrusions protrude into the receiving space. Alternatively, the lower leg frame 6a may have a hollow rectangular tube shape configured to accommodate an outer perimeter of the side support 600a of the connection frame 600. A section of the lower leg frame 6d that has the protrusions may be referred to as a length adjustment drive section or portion.

There may be a receiving space within a hollow space of the bottom portion of the lower leg frame 6d, and protrusions, grooves, or any combination thereof may be provided on inner walls of the hollow rectangular tube. For convenience of description, an example where the notches 613h form indentations or grooves that couple to protrusions of the lower leg frame 6d will be described. The protrusions of the lower leg frame 6d may be formed to protrude into the receiving space.

Embodiments disclosed herein are not limited to the above-described Π-shape or hollow rectangular tube shape, and the lower leg frame 6d and the side support 600a of the connection frame 600 may have other shapes (e.g., cylindrical pipe) such that an outer perimeter of the connection frame 600 is configured to be inserted into a receiving space of the lower leg frame 6d. In yet another alternative embodiment, the third wall 615 may be omitted, and the lower leg frame 6d may instead be inserted into the connection frame 600. In this alternative embodiment, the notches 613h may be formed on inner sides of the first and second walls 613 and 614 to couple to protrusions formed on an outer surface of the lower leg frame 6d. The notches 613h may further only be formed on one of the first or second walls 613 or 614 to couple to corresponding protrusions of the lower leg frame 6d.

For convenience of description, an example where the connection frame 600 is inserted into a receiving space of the lower leg frame 6d will be described. When the connection frame 600 is inserted into the receiving space of the lower leg frame 6d, notches 613h exposed to an outside of the connection frame 600 through the opening 621 may couple to the protrusions that protrude into the inner space of the lower leg frame 6d. When the connection frame 600 is inserted a maximum amount into the receiving space, a maximum number of notches 613h may couple to the protrusions, and a lower calf length of the exoskeleton A created by the connection frame 600 and the lower leg frame 6d may be at a minimum. When the connection frame 600 is inserted a minimum amount into the receiving space, a minimum number of notches 613h may couple to the protrusions, and the lower calf length of the exoskeleton A may be at a maximum.

There may further be a length adjustment drive (e.g., a hydraulic cylinder or linear actuator) provided in the connection frame 600 to drive an insertion of the connection frame 600 into the receiving space of the lower leg frame 6d. Alternatively, the length adjustment drive may be provided in the lower leg frame 6d. The length adjustment drive may be controlled by the main controller or the subcontroller so that the user can automatically adjust the lower calf length. The length adjustment drive may be omitted, in which case the lower calf length may be manually adjusted.

A housing coupling portion or coupler (625 in FIG. 20) that attaches to the ankle support 500 may be formed at a lower end of the frame housing 620. The first connector 514 and the first holder 520 of the ankle body 510 may be inserted into the housing coupler 625 for attachment. The housing coupler 625 may include a fastening portion (not shown), which may be coupled with the second bracket 528. For example, a bolt may secure the housing coupler 625 and the sixth connection hole 539. However, various fastening methods may be applied to the housing coupler 625 and the second bracket 528.

The frame cover 630 may cover a second surface of the skeleton frame 610 opposite the first surface of the skeleton frame 610 such that the skeleton frame 610 may be mounted between the frame housing 620 and the frame cover 630. The frame cover 630 may be formed in a substantially similar curved shape as the base plate 612. Like the base plate 612 and the frame housing 620, the frame cover 630 may include a shape or portion that is guided along the side of the leg of the user and corresponds to the shape of the side support 600a, and a shape or portion that covers the leg of the user and corresponds to the shape of the rear support 600b. The frame cover 630 may cover an opened surface of the frame housing 620. When the frame housing 620 and the frame cover 630 are coupled, an internal space may be formed, which the skeleton frame 610 may occupy.

The frame housing 620 and the frame cover 630 may be formed of a synthetic material that is less rigid than that of the skeleton frame 610. The frame housing 620 and the frame cover 630 may be waterproof or dustproof to prevent a corrosion of the skeleton frame 610, and may be formed of a plastic molding or a synthetic material to reduce weight of the connection frame 600. However, embodiments disclosed are not limited thereto, and the frame housing 620 and the frame cover 630 may be formed of a different material from each other.

Referring to FIGS. 20 to 23, a first axis L1 may pass through a center of the ankle support 500 and may be arranged longitudinally along the ankle support 500. A second axis L2 may pass a center of the side support 600a and may be arranged longitudinally along the side support 600a. Hereinafter, a shape and a structure of the connection frame 600 will be described by using the first axis L1 and the second axis L2.

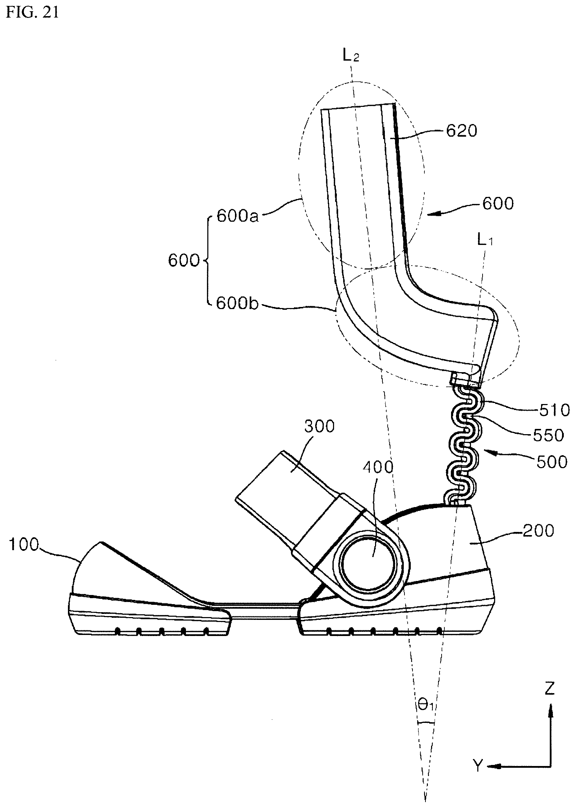

Referring to FIG. 21, when the foot support 7 is viewed from a side, there may be a first angle θ₁ between the first axis L1 and the second axis L2. The first angle θ₁ may be predetermined such that the ankle support 500 does not directly touch or contact a heel or Achilles tendon of the user.

When the user wears the exoskeleton A, a general rear of the foot of the user and the leg of the user may be arranged or aligned on the second axis L2. The ankle support 500 may be inclined rearward by the first angle θ₁ from L2 in order to prevent a direct interference with the exoskeleton A when the ankle of the user moves. The first angle θ₁ may be an acute angle.

The first angle θ₁ between the first axis L1 and the second axis L2 may be freely adjusted or oriented in any direction (e.g., upward, downward, forward, rearward, leftward, and rightward) even while the user wears the exoskeleton A. This structure of the foot support 7 may minimize interference between the ankle support 500 and the ankle of the user while also minimizing restricted movement of the user. The above structure can improve a comfort and an operational convenience of the user. The first angle θ₁ between the first axis L1 and the second axis L2 may be modified depending on a user's body since the ankle support 500 may be formed of an elastic material.

Referring to FIG. 22, when the foot support 7 is viewed from the rear, the first axis L1 and the second axis L2 may be spaced apart from each other by a predetermined distance W1. When viewed from the rear of the foot support 7 of the exoskeleton A, the first axis L1 and the second axis L2 may appear to be parallel.

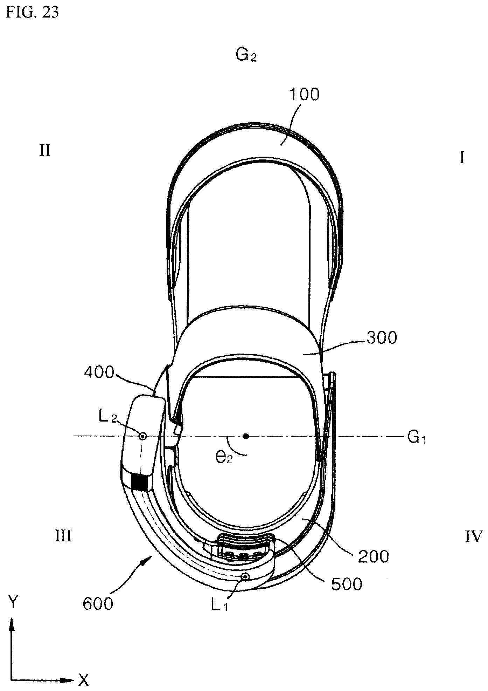

Referring to FIG. 23, the rear support 600b may be bent along a circumference of the leg of the user. The rear support 600b of the connection frame 600 may extend from a center of a rear surface of a lower calf of the user toward an outer side of the user. The rear support 600b may have a predetermined curvature. An arc length and/or radius of curvature of the rear support 600b when viewed from above (FIG. 23) may be larger than that of the second support 200.

The second support 200 may be divided into four areas or sections based on a first guide line G1, which may run in a first direction (e.g., a width direction of the foot support 7) and a second guide line G2, which may run in a longitudinal direction (e.g., a length direction of the foot support 7) perpendicular to G1. The rear support 600b may be arranged in a third quadrant III based on the first guide line G1 and the second guide line G2. Additionally, when the foot support 7 is viewed from above, a second angle θ₂ between a first axis L1 and a second axis L2 may centered on a bottom surface of the foot support 7. The second angle θ₂ may be a right angle or an obtuse angle, but embodiments disclosed herein are not limited thereto.

A shape and a position of the rear support 600b may be variously modified depending on a body structure of the user. The shape of the rear support 600b may be configured to avoid an interference with the ankle bone of the user wearing the exoskeleton A and to minimize physical impact during walking. The rear support 600b may also prevent falls and help to sturdy or balance the user.

An overall shape of the connection frame 600 may reduce fatigue and improve comfort so that the user can use the exoskeleton A for a relatively long time, improving rehabilitation. The shape of the connection frame 600 can minimize a preparation time when the user puts on or takes off the foot support 7. The user can easily detach his or her shoes from the foot support 7 of the exoskeleton A, saving time and improving convenience.

Embodiments disclosed herein may control a wearable assistive device such as an exoskeleton including a foot assembly or foot support to support a free ankle movement. Embodiments disclosed herein may provide a comfortable ankle movement to the user by providing a structure capable of rotating at one or a single point of action and supporting an ankle joint of a human body.

Embodiments disclosed herein may provide a wearable assistive device, e.g., an exoskeleton, including a foot support or foot assembly capable of mitigating an impact with a floor generated at a time of walking after wearing the exoskeleton. That is, embodiments disclosed herein may mitigate the impact with the floor at the time of walking by using a structure and a material capable of absorbing the impact with the floor.

Embodiments disclosed herein may be capable of minimizing an interference between an ankle and the exoskeleton that may occur during walking after or while wearing the exoskeleton. Embodiments disclosed herein may minimize the occurrence of a physical impact between a leg of the user and the exoskeleton during walking, thereby maximizing a rehabilitation effect or an effect of a rehabilitation treatment by the user who uses the exoskeleton.

Embodiments disclosed herein are not limited to the above-mentioned objects or capabilities, and the other objects and the advantages of embodiments disclosed herein which are not mentioned can be understood by the following description, and more clearly understood by the disclosed embodiments. It will be also readily seen that the objects and the advantages of this application may be realized by means indicated in the patent claims and a combination thereof.

The foot support according to embodiments disclosed herein may include an ankle supporting portion or ankle support having an accordion spring formed in a pattern in which a bending is repeated and may be connected to a first and second foot supports. The ankle supporting portion may be formed of a material having a zigzag shape or accordion shape and elasticity, so that an angle can be changed in three dimensions at one point of action. As a result, the ankle supporting portion of this application may allow the user to freely move an ankle. By using a zigzag structure and an elastic material capable of absorbing the impact with the floor, it may be possible to mitigate an impact with the floor at the time of walking, thereby preventing an increase in fatigue of the user.

Further, the foot support may minimize an interference between an ankle and the exoskeleton during walking by including a connecting frame portion or a connection frame that is bent at a side of the leg of the user and extended to a rear surface of the leg or foot of the user. The connection frame may have a shape fixed to the side and rear surface of the leg of the user, and a movement may occur only in the ankle support connected to one end of the connecting frame portion. Therefore, the exoskeleton can prevent a collision between an ankle joint and the connection frame during walking. As a result, embodiments disclosed herein may minimize an impact on the body of the user, thereby improving comfort.

An electric wire or wire to transmit a signal of a pressure sensor may penetrate the zigzag pattern of the rear support in the longitudinal direction. The wire may be provided inside of a stabilizer shaft. As a result, embodiments disclosed herein may simplify an arrangement of the electric wire and prevent the electric wire from being caught or twisted by an external object. The foot support may include a folding portion or bending portion that is bent from the side of the leg of the user and extended to the rear side, thereby minimizing an operation time or preparation time when the user dons or takes off the foot support.

The ankle support may be formed in a pattern in which bending is repeated (for example, the zigzag pattern or accordion pattern), so that the ankle may move freely in three-dimensional direction leg, (a forward-rearward-leftward and rightward-upward and downward direction, or in forward, rearward, leftward, rightward, upward, and downward directions), even in a state in which the user wears the exoskeleton. The user may naturally move the ankle due to the ankle support, and thus, the user can easily change directions and walk. As a result, the degree of freedom of a movement of the user is increased, and restricted movement of the user is minimized. Comfort of the user may also be improved.

The ankle support may be formed in the zigzag pattern and formed of a material having elasticity so as to mitigate the impact with the floor or ground at the time of walking. Therefore, embodiments disclosed herein can mitigate an impact that may be increased in proportion to a weight of the user and the exoskeleton, thereby preventing a body of the user from being immoderate or uncontrollable. Further, a fatigue of the user who uses exoskeleton may be reduced. The user may feel less fatigued even if the user uses the exoskeleton for a long time, and accordingly, rehabilitation treatment can be improved.

Further, the foot support may minimize an interference between the ankle and the exoskeleton during walking by including the connecting frame portion or connection frame, which may be bent at the side of the leg of the user and extended to the rear of the foot support. As a result, it is possible to minimize the physical impact between the leg of the user and the exoskeleton during walking, and prevent the body of the user from being immoderate or uncontrollable.

Embodiments disclosed herein may reduce a production cost of the exoskeleton by minimizing an amount of electric wire required for a signal transmission. Further, an arrangement shape of the electric wire penetrating the ankle supporting portion can act as a design element that improves an aesthetic sense of the foot support.

The foot support of the exoskeleton according to embodiments disclosed herein may include a bending portion or rear support that is bent from the side of the leg of the user and is extended to the rear surface of the foot support, thereby minimizing the time it takes for a user to fix or separate his or her shoes or his or her feet from the exoskeleton. The user may easily fix to and separate from the foot support, and a wearing time or preparation time of the exoskeleton can be reduced. Further, since an operation required to detach the shoes can be simplified, a convenience of the user can be improved.

Embodiments disclosed herein may provide a wearable assistive device or exoskeleton comprising a waist and/or pelvic frame that covers a waist or a pelvis of a user; a leg or leg assembly that extends from the main frame; and a foot assembly or foot support provided at a first end of the leg assembly. The foot support may include a foot support structure or first and second supports to support a foot or a shoe of the user, and an ankle support provided between the leg assembly and the foot support, the ankle support having repeating bends. The ankle support may comprise an ankle body in which a surface of the ankle body is formed in a zigzag or accordion pattern. The ankle body may comprise a pattern member having the repeated bends, a first connector that extends from a first end of the pattern member, and a second connector that extends from a second end of the pattern member.

The repeated bends in the pattern member may repeat along a longitudinal direction of the ankle body, and the first and second connectors may extend in the longitudinal direction such that the ankle support couples the foot support to the leg assembly. The ankle support may comprise a stabilizer shaft that may penetrate the repeated bends and that may be arranged on a center line of the ankle support such that a part of the stabilizer shaft is exposed.

The foot support may further comprise a connection frame having a first end connected to the first end of the leg, and a second end connected to the ankle support such that the connection frame curves as it extends from the leg assembly to the ankle support. The ankle body may include a zigzag pattern, and may be formed of an elastic material. A first holder may be coupled to a first side of the ankle body and the connection frame, and a second holder may be coupled to a second side of the ankle body and a rear of the foot support. The first and second holders may be formed of a different material from each other. The ankle support may be connected to a rear of the foot support. The ankle support may be provided at a location that corresponds to a rear surface of a leg of the user, and the leg assembly of the exoskeleton may be provided at a location that corresponds to a side surface of the leg of the user.

A connection frame may couple the ankle support to the leg assembly of the exoskeleton. A controller may control a drive, motor, or actuator in the leg assembly to provide a force to the user. The foot support may include a first support and a second support slideably coupled to each other, the first support and the second support each having a pressure sensor. Each pressure sensor may be electrically connected to the controller via a wire that penetrates the repeating bends in the ankle support.

Embodiments disclosed herein may provide a wearable assistive device or exoskeleton comprising a main frame that covers a waist or a pelvis of a user; a leg or leg assembly that extends from the main frame; and a foot assembly or foot support provided at a first end of the leg assembly to support a foot or a shoe of the user, wherein the foot assembly may comprise a pattern having repeated folds. The leg assembly may be formed to extend downward along legs, hips, or ilium of the user at an end of the main frame; and the foot assembly may be installed at an end of the leg assembly to support a foot or the shoes of the user. The foot assembly may be formed of an elastic material, and may have a first end formed in a zigzag pattern.

Embodiments disclosed herein may provide a wearable assistive device or exoskeleton comprising a main frame including a belt or waist belt that couples to a waist or a pelvis of a user; a leg assembly that extends along a leg, hips, or ilium of the user from the main frame; and a foot assembly or foot support provided at an end of the leg assembly. The foot support may comprise a foot support structure comprising first and second supports to support a foot or a shoe of the user, an ankle support connected to the foot support, and a connection frame having a first end connected to the leg assembly and a second end connected to the ankle support, such that the connection frame bends from the first end to the second end.

The connection frame may include a side portion extending from the leg assembly at the first end such that the side portion is provided at a position corresponding to a side surface of the leg of the user, and may include a bent portion that is bent from the side portion and is connected to the ankle support at the second end such that the bent portion is provided at a position corresponding to a rear surface of the leg of the user.

The connection frame may comprise a skeleton frame provided inside the side portion and the bent portion, a frame housing that covers a first surface of the skeleton frame, and a frame cover that covers a second surface of the skeleton frame. A first axis provided along a longitudinal direction of the ankle support may be spaced apart from a second axis provided along a longitudinal direction of the side portion by a predetermined distance. The first axis and the second axis may form an acute angle when viewed from a side of the foot assembly.

Since various substitutions, changes, and modifications can be made within the scope that does not deviate the technical idea of this application for those skilled in the art to which this application pertains, embodiments disclosed herein are not limited by the above-mentioned embodiments and the accompanying drawings.

It will be understood that when an element or layer is referred to as being "on" another element or layer, the element or layer can be directly on another element or layer or intervening elements or layers. In contrast, when an element is referred to as being "directly on" another element or layer, there are no intervening elements or layers present. As used herein, the term "and/or" includes any and all combinations of one or more of the associated listed items.

It will be understood that, although the terms first, second, third, etc., may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section could be termed a second element, component, region, layer or section without departing from the teachings of the present invention.

Spatially relative terms, such as "lower", "upper" and the like, may be used herein for ease of description to describe the relationship of one element or feature to another element(s) or feature(s) as illustrated in the figures. It will be understood that the spatially relative terms are intended to encompass different orientations of the device in use or operation, in addition to the orientation depicted in the figures. For example, if the device in the figures is turned over, elements described as "lower" relative to other elements or features would then be oriented "upper" relative the other elements or features. Thus, the exemplary term "lower" can encompass both an orientation of above and below. The device may be otherwise oriented (rotated 90 degrees or at other orientations) and the spatially relative descriptors used herein interpreted accordingly.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Embodiments of the disclosure are described herein with reference to cross-section illustrations that are schematic illustrations of idealized embodiments (and intermediate structures) of the disclosure. As such, variations from the shapes of the illustrations as a result, for example, of manufacturing techniques and/or tolerances, are to be expected. Thus, embodiments of the disclosure should not be construed as limited to the particular shapes of regions illustrated herein but are to include deviations in shapes that result, for example, from manufacturing.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Any reference in this specification to "one embodiment," "an embodiment," "example embodiment," etc., means that a particular feature, structure, or characteristic described in connection with the embodiment is included in at least one embodiment of the invention. The appearances of such phrases in various places in the specification are not necessarily all referring to the same embodiment. Further, when a particular feature, structure, or characteristic is described in connection with any embodiment, it is submitted that it is within the purview of one skilled in the art to effect such feature, structure, or characteristic in connection with other ones of the embodiments.

## Claims

1. A wearable assistive device, comprising:
a main frame (4) configured to support a waist or a pelvis;
a leg assembly (6) extending downward from an end of the main frame (4); and
a foot support (7) installed at an end of the leg assembly (6) to support a foot, wherein the foot support (7) includes an Achilles extension (512) having an accordion spring configured to extend behind a rear of an Achilles tendon.

2. The wearable assistive device of claim 1, wherein a stabilizer shaft (550) penetrates the accordion spring in a longitudinal direction such that the stabilizer shaft (550) is partially exposed to an outside.

3. The wearable assistive device of claim 1, or 2, wherein the accordion spring is formed of an elastic material, and the Achilles extension (512) includes a first connector (514) and a second connector (516) formed at top and bottom ends of the accordion spring.

4. The wearable assistive device of claim 3, wherein the first connector (514) couples to a first protuberance (525), the first protuberance (525) extending from a first bracket (528) that is coupled to the leg assembly (6), and wherein the second connector (516) couples to a second protuberance (535), the second protuberance (535) extending from a second bracket (538) that is coupled to a rear support (200) of the foot support (7) that supports a heel.

5. The wearable assistive device of claim 4, wherein the first and second brackets (528; 538) are formed of a different material from each other.

6. A wearable assistive device, comprising:
a main frame (4) configured to fit on a waist or a pelvis;
a leg assembly (6) that extends from the main frame (4);
a foot support (7) configured to support a foot or a shoe,
an ankle support (500) connected to the foot support (7), and
a connection frame (600) having a first end connected to the leg assembly (6) and a second end connected to the ankle support (500), wherein the connection frame (600) is bent from a side of the leg to a rear of the leg.

7. The wearable assistive device of claim 6, wherein the connection frame (600) includes a side support (600a) extending from the leg assembly (6) at the first end such that the side support (600a) is provided at a position aligning with the side of the leg, and a rear support (600b) that is bent from the side support (600a) and is connected to the ankle support (500) at the second end such that the rear support (600b) is provided behind the rear of the leg.

8. The wearable assistive device of claim 7, wherein the connection frame (600) comprises:
a skeleton frame (610) provided inside the side support (600a) and the rear support (600b),
a housing (620) that covers a first surface of the skeleton frame (610), and
a cover (630) that covers a second surface of the skeleton frame (610).

9. The wearable assistive device of claim 7, or 8, wherein a first axis (L1) provided along a longitudinal direction of the ankle support (500) deviates from a second axis (L2) provided along a longitudinal direction of the side support (600a) by a predetermined angle.

10. The wearable assistive device of claim 9, wherein the predetermined angle is an acute angle when viewed from a side of the foot support (7).

11. A wearable assistive device, comprising:
a leg assembly (6) configured to be secured to a leg;
a foot support (7) configured to be secured to a foot;
an ankle support (500) extending between the foot support (7) and the leg assembly (6) that includes an Achilles extension (512);
wherein the Achilles extension (512) is made of a flexible material and is configured to twist around a longitudinal axis (L1) of the ankle support (500) and rotate relative to a point where the ankle support (500) connects to the leg assembly (6), allowing eversion motion of the foot support (7), inversion motion of the foot support (7), plantar flexion motion of the foot support (7), dorsiflexion motion of the foot support (7), and rotation about a longitudinal axis of the Achilles extension (512).

12. The wearable assistive device of claim 11, wherein the Achilles extension (512) includes an accordion spring that can expand and contract.

13. The wearable assistive device of claim 11, or 12, further including a waist frame (5) configured to be secured to a waist, wherein the leg assembly (6) connects the waist frame (5) to the ankle support (500).

14. The wearable assistive device of claim 11, 12. or 13, wherein the ankle support (500) extends from a rear of the foot support (7) behind an Achilles tendon, the leg assembly (6) extends along a side of the leg such that it does not interfere with a bending of the knee or hip, and wherein the wearable assistive device further includes a connection frame (600) that couples the ankle support (500) to the leg assembly (6) such that the connection frame (600) is curved.

15. The wearable assistive device of any one of claims 11 to 14, further including a controller that controls a drive in the leg assembly (6) to rotate at least one joint of the leg assembly (6).
